# EUROPÄISCHE PATENTSCHRIFT

(11) **EP 4 511 092 B1**
(45) Veröffentlichungstag und Bekanntmachung des Hinweises auf die Patenterteilung: **11.02.2026**
(21) Anmeldenummer: 23721317.8
(22) Anmeldetag: 19.04.2023
(51) Int. Cl.: A61M 13/00

(54) **INSUFFLATIONSVORRICHTUNG MIT SCHONMODUS**
INSUFFLATION APPARATUS HAVING A GENTLE MODE
APPAREIL D'INSUFFLATION AYANT UN MODE DOUX

(30) Priorität: 21.04.2022 DE 102022001374
(43) Veröffentlichungstag der Anmeldung: 26.02.2025
(73) Patentinhaber: W.O.M. World of Medicine GmbH, 10587 Berlin (DE)
(72) Erfinder: GRUNDMANN, Julia, 13187 Berlin (DE); HILDEBRAND, Matthias, 10559 Berlin (DE)
(74) Vertreter: Gulde & Partner
(86) Internationale Anmeldenummer: PCT/EP2023/060170
(87) Internationale Veröffentlichungsnummer: WO 2023/203093

(56) Entgegenhaltungen:
- WO-A2-2007/050516
- US-A1- 2010 106 080
- US-B2- 9 561 335

## Beschreibung

Die vorliegende Erfindung betrifft einen Insufflator mit einer neuartigen Eingabeverarbeitung für den Sollwert des Druckes in der Körperhöhle. Durch die neue Vorrichtung ist es möglich, den Druck in der Körperhöhle bei ausreichender Aufdehnung als Solldruck vorzugeben und in die bisherige Regelung zu übernehmen um möglichst patientenschonend zu arbeiten.

### Hintergrund und Stand der Technik

Insufflatoren sind in mannigfaltiger Ausführung aus dem Stand der Technik bekannt. Insufflatoren weisen üblicherweise einen Schlauch auf, durch den ein medizinisches Gas in eine Körperhöhle (z. B. ein Abdomen) eingeleitet wird. Das Gas erzeugt einen Überdruck, welcher die Körperhöhle aufdehnt, damit ausreichend Platz für die visuelle Inspektion bzw. den therapeutischen Eingriff bzw. die medizinische Prozedur besteht. Optionale Ausführungsformen ermöglichen das Absaugen des Gases aus der Körperhöhle über einen zweiten Schlauch. Derartige Ausführungsformen werden insbesondere bei therapeutischen Eingriffen mittels Elektrochirurgie oder Laser genutzt, um gesundheitsschädliche Rauchgase abzuführen und zu filtern, sowie die Sicht beim Eingriff zu gewährleisten.

In der Praxis hat es sich als schwierig herausgestellt, den Druck in der Körperhöhle patientenabhängig über den Verlauf der gesamten Prozedur zu minimieren. Durch eine minimale Druckerhöhung, wie es der natürlichen Situation ja entsprechen würde, ist eine Überlastung oder Verletzung von Strukturen in der Körperhöhle ausgeschlossen bzw. können sogar negative Effekte durch Einwirken über den Druck in der Körperhöhle auf den Blutdruck des Patienten verhindert werden.

Gleichzeitig muss die notwendige Aufdehnung erfolgen, um die Prozedur ohne hohen Aufwand beim Operateur und Risiko durch Instrumentenbewegungen oder fehlende Sicht für den Patienten durchführen zu können.

Die Vorgabe, möglichst wenig Druck durch das Aufdehnungsmedium einzubringen und gleichzeitig den erforderlichen Bewegungs- und Sichtraum zu schaffen, um die medizinische Prozedur durchführen zu können, wird üblicherweise durch eine Einstellung eines Vorgabe- oder Solldruckes für das Regelsystem im Insufflator vorgenommen.

Eine patientenindividuelle Einstellung erfordert durch Ausprobieren und Herantasten an den optimalen Sollwert viel Zeit des Operateurs oder Bedienpersonals. Alternativ muss der Operateur seine Erfahrungswerte einbringen, so dass hier vielfältige Einflüsse die sichere Einschätzung beeinflussen und letztlich für den Patienten keine zuverlässige Schonung seines Körpers gewährleisten.

Der Weg die minimale Aufdehnung über eine einfache Einleitung von nur wenig Aufdehnungsmedium zu realisieren ist nicht zielführend, da durch Instrumentenwechsel und Leckagen ständig Aufdehnungsmedium verloren geht und eine adäquate Menge nachzufüllen für eine einfache Zuleitung von Medium dem Bediener viel Aufwand macht. Daher ist es üblich eine Regelung für die Menge des Aufdehnungsmediums in der Körperhöhle einzusetzen, wobei die Stellgröße üblicherweise der Druck in der Körperhöhle ist (EP3049136B1).

Eine Vorgabe anhand der Auswahl im Gerät von sogenannten Indikationen, also Voreinstellungen, ob es sich beim Patienten um eine übergewichtige Person, eine sportliche Person mit festem Bindegewebe oder ein Kind handelt, kann nur in groben Kategorien die Sollwerte vorgeben.

Verkompliziert wird die Situation, wenn nicht nur eine Aufdehnung, sondern auch eine Absaugung während der medizinischen Prozedur erfolgen soll. Hierbei ist Aufgabe der Druckregelung für die Körperhöhle die Leckagen auszugleichen und einen Überdruck zu vermeiden. Da das abgesaugte Aufdehnungsmedium üblicherweise durch einen Filter wieder zugeführt wird, ist eine Pumpe im Absaugungs- bzw. Entrauchungskreis enthalten, die einen zweiten Aktor im Regelsystem darstellt, während der Rauchgasfilter eine weitere Störgröße liefert, u.a. durch seine im Laufe der Zeit veränderliche Durchlässigkeit. Hierzu wird typischerweise ein etwas erhöhter Solldruck akzeptiert.

Etabliert haben sich daher Ausführungsformen, bei denen über die Bedienoberfläche des Gerätes der Solldruck stufenweise erhöht oder verringert werden kann.

Weiterer Stand der Technik wird durch die folgenden Dokumente gebildet:
- US2010/106080 A1 beschreibt eine Insufflationsvorrichtung welches einen ersten Druck in einer ersten Körperhöhle eines Patienten und einen zweiten Druck in einer zweiten Körperhöhle des Patienten misst. Die Vorrichtung regelt den Druck eines vorbestimmten Gases auf der Grundlage des gemessenen ersten und zweiten Drucks innerhalb der ersten und zweiten Körperhöhle.
- US9,561,335 B2 offenbart eine Insufflationsvorrichtung mit Steuergerät zum Erfassen eines Druckniveaus des aufblähenden Mediums innerhalb eines Organs eines Patienten, wobei das Steuergerät so konfiguriert ist, dass es einem Bediener signalisiert, ein Bild des Organs zu erfassen, wenn das Druckniveau innerhalb eines vorbestimmten Druckbereichs für eine im Voraus festgelegte Zeitspanne liegt.
- WO2007/050516 A2 offenbart ein System zur Steuerung der Zufuhr eines Aufweitungsmediums (z. B. eines Insufflationsgases) einer endoskopischen Vorrichtung, um eine übermäßige Entlüftung und/oder Verschwendung von Aufweitungsmedien zu verhindern. Das System passt einen Zufuhrparameter des Aufblasmediums auf der Grundlage des erfassten Druckniveaus an.

### Erfindungsgemäße Lösung

Die Erfindung ist in den angehängten Ansprüchen definiert. Um die oben genannten Nachteile bisheriger Solldruckvorgaben zu überwinden, hat sich die Aufgabe gestellt, eine Eingabemöglichkeit vorzugeben, die einen Sollwert ohne Fehleingaben oder zusätzlichen Aufwand in das Regelsystem des Insufflators übernimmt.

Die vorliegende Erfindung betrifft daher einen Insufflator für die minimalinvasive Insufflator für die minimal-invasive Chirurgie, enthaltend
a) Gasanschluss mit Proportionalventil,
b) einen ersten Trokar
c) eine Gaszuführleitung für Gas aus dem Gasanschluss mit Druckmessung, Filter und Anschluss an den ersten Trokar zum Insufflieren von Gas in eine Kavität, wobei der Gasstrom durch einen Massendurchflussregler im Bereich 0-50 L/min regelbar ist,
d) optional mindestens einen Gasflusssensor der zur Bestimmung des Gasflusses in die Kavität eingerichtet ist,
e) mindestens einen Drucksensor der zur Bestimmung des Druckes in der Kavität eingerichtet ist,
f) optional eine Absaugvorrichtung mit regelbarer Absaugleistung,
g) einen zweiten Trokar zum Gasauslass aus der Kavität optional mit Absaugschlauch, angeschlossen an die Absaugvorrichtung mit regelbarer Absaugleistung,
h) eine elektronische Regelungseinheit mit mindestens einem Speicher zum Abspeichern mindestens eines ersten Solldruckes und eines zweiten Solldruckes,
i) eine erste Eingabevorrichtung,
j) eine zweite Eingabevorrichtung.
k) eine Anzeigeeinrichtung

wobei die elektronische Regelungseinheit die Regelung des Druckes in der Kavität durch Änderung des Gaszuflusses vornimmt,
wobei mittels der ersten Eingabevorrichtung der elektronischen Regelungseinheit vor Beginn der Insufflation ein erster Solldruck in der Kavität vorgegeben wird,
wobei durch Betätigung der zweiten Eingabevorrichtung vor Erreichen des ersten Solldrucks der elektronischen Regelungseinheit den zum Zeitpunkt der Betätigung der zweiten Eingabevorrichtung herrschenden Druck als zweiten Solldruck vorgegeben werden kann,
wobei der zweite Solldruck von der elektronischen Regelungseinheit für die weitere Insufflation gehalten wird.

Weitere Ausgestaltungen der Offenbarung inklusive der dazugehörigen Betriebsverfahren werden nachfolgend beschrieben und sind teilweise Gegenstand von Neben- und Unteransprüchen.

### Grundzüge der Offenbarung einfache Ausführungsformen

Die Offenbarung umfasst eine Methode den Druck in der Körperhöhle langsam zu erhöhen und auf einen geeigneten Input zu reagieren, indem der aktuell erreichte Wert als Solldruckwert in das Regelsystem des Insufflators übernommen wird. Typischerweise wird die elektronische Regelungseinheit zu Beginn der Insufflation einen möglichst hohen Gasfluss einstellen, um den eingestellten ersten Solldruck möglichst schnell zu erreichen.

Im neuartigen Schonmodus stoppt der Anwender die Aufdehnung, sobald sie groß genug für die Durchführung der medizinischen Prozedur ist. Die Methode sieht vor, den Druck in der Körperhöhle langsam zu erhöhen und dabei auf einen geeigneten Input zu warten. Damit wird die optimale Aufdehnung für den konkreten Einzelfall durch den Anwender festgelegt und gleichzeitig sichergestellt, dass die Körperhöhle nur so weit aufgedehnt wird, wie unbedingt notwendig. Dieser durch die Eingabe (Input) bestätigte Druckwert (Solldruck) wird somit der neue Standardsollwert für diese medizinische Prozedur. Weiterhin kann jederzeit der Druckwert verändert werden, wie es im Stand der Technik beschrieben ist.

Erfolgt kein Input, also wird die Aufdehnung nicht aktiv beeinflusst, wird wie bisher üblich der im Gerät für die ausgewählte Indikation vorgesehene Solldruck genutzt. Ist keine Indikation im Gerät auszuwählen, wird der vorgesehene oder zuletzt gewählte Solldruck in das Regelsystem übernommen. Der geeignete Input erfolgt üblicherweise über eine Abfrage/Eingabe auf den angeschlossenen Bedienoberflächen. Weitere Optionen für die Einstellung von Parametern (u.a. auch des Solldruckes) sind ein Input von einem Knopf zum Weißabgleich auf dem Kamerakopf, über ein Fußpedal, über eine Sprachsteuerung sowie über andere mit dem Gerät verbunden Eingabegeräte bzw. Fernbedienungen.

Üblicherweise wird zu Beginn der Insufflation der Gasfluss möglichst hoch gewählt, damit die Operationsvorbereitung nicht zu lange dauert. Die initiale Insufflation kann bei erwachsenen Patienten durch einen Gasfluss von 3 L/min erfolgen. Der in der Kavität (z.B. Abdomen) angestrebte Druck beträgt dabei ca. 15 mmHg. Der angestrebte Druck wird bei dem angegebenen Gasfluss nach ca. 1 Minute erreicht (Kavitätsvolumen 3 L bei 15 mmHg).

Für kleinere Kavitäten (z.B. Enddarm) beträgt der initiale Gasfluss 3 L/min. Der angestrebte Druck von 12 mmHg wird nach ca. 4 s erreicht (Kavitätsvolumen 200 ml bei 12 mmHg).

### Weitere Ausführungsformen der Offenbarung

In weiteren Ausführungsformen der Offenbarung kann, nachdem eine initiale Aufdehnung erfolgt ist, die gleiche Methode genutzt werden, um die Aufdehnung zu korrigieren.

Hierzu wird eine Sequenz gestartet, in der keine medizinische Prozedur durchgeführt wird und zunächst der Druck in der Körperhöhle verringert wird um einen definierten Wert, oder über die gleiche Methode den Druck zu senken bis ein Input kommt. Auch hier ist die Grenze, sofern kein Input kommt, ein im Gerät festgelegter Wert.

Anschließend wird der Druck in der Körperhöhle langsam erhöht, bis der Benutzer einen Input mit den oben bereits beschriebenen Möglichkeiten gibt. Erfolgt kein Input wird die im Gerät - wie bereits oben beschrieben - hinterlegte Größe verwendet.

In einer weiteren Ausführungsform wird die Sequenz gestartet ohne den Druck vorher zu verringern.

In einer weiteren Ausführungsform wird der Druck während der Rauchgasabsaugung ebenfalls mit oder ohne vorherige Verringerung des Körperhöhlendruckes erhöht und der durch den Input bestimmt Wert wird als Solldruck für den Modus der Rauchgasabsaugung festgelegt und nach Abschaltung wird wieder der zuletzt für den normalen Modus gewählte Solldruck zur Regelung genutzt. In diesem Fall kann die Druckerhöhung bis zum neuen Solldruck langsamer, das heißt mit einem geringeren Gasfluss, vorgenommen werden, beispielsweise mit einem Gasfluss, der 75%, 50% oder 25% des initialen Gasflusses beträgt.

Alle diese Kombinationen gelten als erfinderisch und bieten eine einfache Methode um ohne große Interaktion mit Personen außerhalb des sterilen Bereiches eine Anpassung des Solldruckwertes durch den Bediener, der üblicherweise auch die medizinische Prozedur durchführt, zu ermöglichen im Sinne einer maximalen Patientenschonung.

### Beschreibung der Figuren

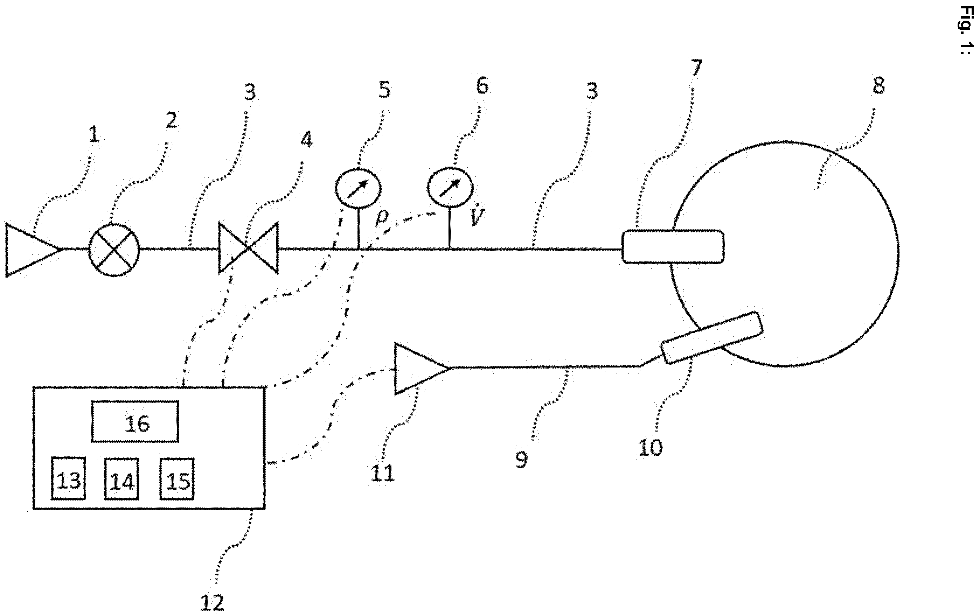

Die Erfindung wird grundsätzlich in Figur 1 dargestellt.

Die in der Figur 1 verwendeten Referenzzeichen haben folgende Bedeutung:
(1) Gasanschluss
(2) Proportionalventil
(3) Gaszuführleitung
(4) Volumenstromregelung
(5) Drucksensor
(6) Volumenstromsensor (optional)
(7) erster Trokar
(8) Kavität
(9) Absaugschlauch
(10) zweiter Trokar
(11) Absaugvorrichtung mit regelbarer Absaugleistung
(12) erste Eingabevorrichtung
(13) zweite Eingabevorrichtung
(14) dritte Eingabevorrichtung
(15) Betätigungsvorrichtung (optional)
(16) Anzeigeeinrichtung

## Patentansprüche

1. Insufflator für die minimal-invasive Chirurgie, enthaltend
a) Gasanschluss (1) mit Proportionalventil (2),
b) einen ersten Trokar (7)
c) eine Gaszuführleitung (3) für Gas aus dem Gasanschluss mit Druckmessung, Filter und Anschluss an den ersten Trokar (7) zum Insufflieren von Gas in eine Kavität (8), wobei der Gasstrom durch einen Massendurchflussregler im Bereich 0-50 L/min regelbar ist,
d) optional mindestens einen Gasflusssensor (6) der zur Bestimmung des Gasflusses in die Kavität (8) eingerichtet ist,
e) mindestens einen Drucksensor (5) der zur Bestimmung des Druckes in der Kavität (8) eingerichtet ist,
f) optional eine Absaugvorrichtung (11) mit regelbarer Absaugleistung,
g) einen zweiten Trokar (10) zum Gasauslass aus der Kavität (8) optional mit Absaugschlauch (9), angeschlossen an die Absaugvorrichtung (11) mit regelbarer Absaugleistung,
h) eine elektronische Regelungseinheit mit mindestens einem Speicher zum Abspeichern mindestens eines ersten Solldruckes und eines zweiten Solldruckes,
i) eine erste Eingabevorrichtung (12),
j) eine zweite Eingabevorrichtung (13),
k) eine Anzeigeeinrichtung (16)
wobei die elektronische Regelungseinheit die Regelung des Druckes in der Kavität (8) durch Änderung des Gaszuflusses vornimmt,
wobei mittels der ersten Eingabevorrichtung (12) der elektronischen Regelungseinheit vor Beginn der Insufflation ein erster Solldruck in der Kavität (8) vorgegeben wird,
wobei durch Betätigung der zweiten Eingabevorrichtung (13) vor Erreichen des ersten Solldrucks der elektronischen Regelungseinheit den zum Zeitpunkt der Betätigung der zweiten Eingabevorrichtung herrschenden Druck als zweiten Solldruck vorgegeben werden kann,
wobei der zweite Solldruck von der elektronischen Regelungseinheit für die weitere Insufflation gehalten wird.

2. Insufflator gemäß Anspruch 1, wobei die elektronische Regelungseinheit durch erneutes Betätigen der zweiten Eingabevorrichtung (13) den ersten Solldruck in der Kavität (8) durch Änderung des Gasflusses einstellt.

3. Insufflator gemäß Anspruch 1, wobei die elektronische Regelungseinheit eine dritte Eingabevorrichtung (14) aufweist,
wobei die elektronische Regelungseinheit durch Betätigen der dritten Eingabevorrichtung (14) den Druck in der Kavität durch Änderung des Gasflusses verringert,
wobei das medizinische Personal durch erneutes Betätigen der zweiten (13) oder der dritten Eingabevorrichtung (14) den zum Zeitpunkt der Betätigung der zweiten oder dritten Eingabevorrichtung herrschenden Druck als dritten Solldruck vorgeben kann,
wobei der dritte Solldruck von der elektronischen Regelungseinheit für die weitere Insufflation gehalten wird.

4. Insufflator gemäß mindestens einem der Ansprüche 1-3, wobei die zweite oder die dritte Eingabevorrichtung ausgewählt wird aus:
Knopf zum Weißabgleich auf dem Kamerakopf,
Fußpedal,
Sprachsteuerung,
Fernbedienung.

5. Insufflator gemäß Anspruch 1, wobei der Gasfluss zu Beginn der Insufflation 1 - 5 L/min beträgt.

6. Insufflator gemäß Anspruch 2, wobei der Gasfluss nach erneuter Betätigung der zweiten Eingabevorrichtung (13) 1 - 2,5 L/min beträgt.

## Claims

1. An insufflator for minimally invasive surgery, comprising
a) a gas connection (1) with a proportional valve (2),
b) a first trocar (7),
c) a gas supply line (3) for gas from the gas connection with a pressure measurement, filter, and connection to the first trocar (7) for insufflating the gas into a cavity (8), wherein the gas flow is adjustable by a mass flow controller in a range of 0-50 L/min,
d) optionally at least one gas flow sensor (6) configured to determine the gas flow into the cavity (8),
e) at least one pressure sensor (5) configured to determine the pressure in the cavity (8),
f) optionally an extraction device (11) with an adjustable extraction capacity,
g) a second trocar (10) for gas outlet from the cavity (8), optionally with an extraction hose (9) connected to the extraction device (11) with an adjustable extraction capacity,
h) an electronic control unit with at least one memory for storing at least one first setpoint pressure and one second setpoint pressure,
i) a first input device (12),
j) a second input device (13),
k) a display device (16),
wherein the electronic control unit regulates the pressure in the cavity (8) by changing the gas flow,
wherein by means of the first input device (12) of the electronic control unit, a first setpoint pressure in the cavity (8) is specified before the start of insufflation,
wherein by activating the second input device (13) before reaching the first setpoint pressure, the pressure prevailing at the time of actuating the second input device can be specified as the second setpoint pressure, the second setpoint pressure being maintained by the electronic control unit for further insufflation.

2. The insufflator according to claim 1, wherein the electronic control unit sets the first setpoint pressure in the cavity (8) by changing the gas flow through re-activation of the second input device (13).

3. The insufflator according to claim 1, wherein the electronic control unit comprises a third input device (14),
wherein the electronic control unit reduces the pressure in the cavity by changing the gas flow through activation of the third input device (14),
wherein medical personnel can, by reactivating the second input device (13) or the third input device (14), specify the pressure prevailing at the time of activating the second or third input device as a third setpoint pressure,
wherein the third setpoint pressure is maintained by the electronic control unit for further insufflation.

4. The insufflator according to at least one of claims 1-3, wherein the second or third input device is selected from:
a white balance button on a camera head,
a foot pedal,
a voice control,
a remote control.

5. The insufflator according to claim 1, wherein the gas flow at the start of insufflation is 1-5 L/min.

6. The insufflator according to claim 2, wherein the gas flow after re-activating the second input device (13) is 1-2.5 L/min.

## Revendications

1. Insufflateur pour la chirurgie mini-invasive, comprenant
a) un raccord de gaz (1) avec une vanne proportionnelle (2),
b) un premier trocart (7),
c) une conduite d'alimentation en gaz (3) pour le gaz provenant du raccord de gaz, avec mesure de pression, filtre et raccordement au premier trocart (7) pour insuffler du gaz dans une cavité (8), dans laquelle le débit de gaz est réglable au moyen d'un régulateur de débit massique dans la plage de 0 à 50 L/min,
d) en option, au moins un capteur de débit de gaz (6) configuré pour déterminer le débit de gaz vers la cavité (8),
e) au moins un capteur de pression (5) configuré pour déterminer la pression dans la cavité (8),
f) en option, un dispositif d'aspiration (11) avec puissance d'aspiration réglable,
g) un deuxième trocart (10) pour l'évacuation du gaz hors de la cavité (8), en option avec un tuyau d'aspiration (9), raccordé au dispositif d'aspiration (11) avec puissance d'aspiration réglable,
h) une unité électronique de régulation avec au moins une mémoire destinée à mémoriser au moins une première pression de consigne et une deuxième pression de consigne,
i) un premier dispositif de saisie (12),
j) un deuxième dispositif de saisie (13),
k) un dispositif d'affichage (16),
dans lequel l'unité de régulation électronique assure la régulation de la pression dans la cavité (8) en modifiant l'alimentation en gaz,
dans lequel une première pression de consigne dans la cavité (8) est prédéfinie, avant le début de l'insufflation, au moyen du premier dispositif de saisie (12) de l'unité de régulation électronique,
dans lequel l'actionnement du deuxième dispositif de saisie (13) avant que la première pression de consigne ne soit atteinte permet de prédéfinir comme deuxième pression de consigne la pression existant au moment de l'actionnement du deuxième dispositif de saisie, dans lequel la deuxième pression de consigne est maintenue par l'unité électronique de régulation pour la suite de l'insufflation.

2. Insufflateur selon la revendication 1, dans lequel l'unité de régulation électronique règle la première pression de consigne dans la cavité (8) en modifiant le débit de gaz par une nouvelle action sur le deuxième dispositif de saisie (13).

3. Insufflateur selon la revendication 1, dans lequel l'unité électronique de régulation comprend un troisième dispositif de saisie (14),
dans lequel l'unité électronique de régulation réduit la pression dans la cavité en modifiant le débit de gaz par actionnement du troisième dispositif de saisie (14),
dans lequel le personnel médical peut, par un nouvel actionnement du deuxième (13) ou du troisième dispositif de saisie (14), définir la pression existant au moment de l'actionnement du deuxième ou du troisième dispositif de saisie comme troisième pression de consigne,
dans lequel la troisième pression de consigne est maintenue par l'unité électronique de régulation pour la poursuite de l'insufflation.

4. Insufflateur selon au moins l'une des revendications 1 à 3, dans lequel le second ou le troisième dispositif de saisie est choisi parmi :
un bouton de balance des blancs sur la tête de caméra,
une pédale,
une commande vocale,
une télécommande.

5. Insufflateur selon la revendication 1, dans lequel le débit de gaz au début de l'insufflation est de 1 à 5 L/min.

6. Insufflateur selon la revendication 2, dans lequel le débit de gaz après un nouvel actionnement du deuxième dispositif de saisie (13) est de 1 à 2,5 L/min.
